# FASCICULE DE BREVET EUROPEEN

(11) **EP 0 962 223 B1**
(45) Date de publication et mention de la délivrance du brevet: **11.02.2004**
(21) Numéro de dépôt: 99400948.8
(22) Date de dépôt: 19.04.1999
(51) Int. Cl.: A61K 7/48

(54) **Utilisation de l'acide cinnamique ou d'au moins l'un de ses dérivés dans une composition destinée à favoriser la desquamation de la peau, et composition le comprenant**
Verwendung von Zimtsäure oder mindestens einem ihrer Derivate in einer Zusammensetzung, die dazu vorgesehen ist, die Abschuppung der Haut zu fördern, und sie enthaltende Zusammensetzung
Use of cinnamic acid and it's derivatives to stimulate desquamation of the skin and compositions containing it

(30) Priorité: 12.05.1998 FR 9805967
(43) Date de publication de la demande: 08.12.1999
(73) Titulaire: L'OREAL, 75008 Paris (FR)
(72) Inventeur: Breton, Lionel, 78000 Versailles (FR); Liviero, Christel, 75008 Paris (FR)
(74) Mandataire: Tezier Herman, Béatrice

(56) Documents cités:
- EP-A- 0 664 290
- EP-A- 0 716 847
- FR-A- 2 714 600
- FR-A- 2 734 478
- FR-A- 2 761 884
- US-A- 5 093 109
- PATENT ABSTRACTS OF JAPAN vol. 017, no. 674 (C-1140), 10 décembre 1993 (1993-12-10) & JP 05 221845 A (HISAMITSU PHARMACEUT CO INC), 31 août 1993 (1993-08-31)
- "Hair rinsing composition contains hops, nettle and chestnut extracts, safflower concentrate, sunflower oil cake protein production byproduct and isopropyl palmitate" WPI WORLD PATENT INFORMATION DERWENT, vol. 2, no. 88, XP002113672 & SU 1 311 734 A23 mai 1987 (1987-05-23)
- PATENT ABSTRACTS OF JAPAN vol. 017, no. 055 (C-1023), 3 février 1993 (1993-02-03) & JP 04 266807 A (SOUKEN:KK), 22 septembre 1992 (1992-09-22)
- PATENT ABSTRACTS OF JAPAN vol. 018, no. 118 (C-1172), 25 février 1994 (1994-02-25) & JP 05 310526 A (EISAI CO LTD), 22 novembre 1993 (1993-11-22)

## Description

L'invention concerne l'utilisation en tant que composé actif dans une composition d'une quantité efficace d'acide cinnamique ou d'au moins l'un de ses dérivés, l'acide cinnamique ou la composition étant destinés à favoriser la desquamation de la peau et/ou stimuler le renouvellement épidermique. Elle se rapporte aussi à un procédé de traitement non thérapeutique de la peau destiné à favoriser la desquamation et/ou stimuler le renovellement épidermique.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les coméocytes qui sont des cellules mortes qui s'éliminent par desquamation. Cette perte en surface est compensée par la migration de cellules de l'assise basale vers la surface de l'épiderme. Il s'agit du renouvellement perpétuel de la peau. Une élimination forcée de la couche cornée accélère le renouvellement et permet de lutter contre le vieillissement. Dans le même temps ces cellules poursuivent leur différenciation dont le dernier stade est le coméocyte. Il s'agit en fait de cellules mortes qui constituent la dernière couche de l'épiderme, c'est à dire la couche la plus externe encore appelée *stratum corneum.*

Le vieillissement cutané qui résultent des effets sur la peau de facteurs intrinsèques ou extrinsèques, se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté et par l'apparition de télangiectasies.

Certains de ces signes du vieillissement sont plus particulièrement liés au vieillissement intrinsèque ou physiologique, c'est-à-dire au vieillissement « normal » lié à l'âge ou chronobiologique, alors que d'autres sont plus spécifiques du vieillissement extrinsèque, c'est-à-dire du vieillissement provoqué d'une manière générale par l'environnement ; il s'agit plus particulièrement du photovieillissement dû à l'exposition au soleil, à la lumière ou à tout autre rayonnement, naturels ou artificiels.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène.

On connaît dans l'art antérieur divers agents destinés à lutter contre le vieillissement cutané.

Ainsi, le brevet US-A-4603146 décrit l'emploi d'acide rétinoïque et de ses dérivés dans des compositions cosmétiques, en vue de lutter contre le vieillissement cutané.
Par ailleurs, de nombreux brevets et publications (voir par exemple la demande EP-A-413528) ainsi que de nombreuses compositions cosmétiques du commerce enseignent l'emploi des α-hydroxy-acides comme l'acide lactique, l'acide glycolique ou encore l'acide citrique pour traiter le vieillissement cutané.

On connaît enfin les bêta-hydroxy-acides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau en favorisant la desquamation, c'est-à-dire l'élimination des cellules « mortes » situées à la surface de la couche cornée de l'épiderme. Cette propriété "desquamante" est aussi appelée, souvent à tort, propriété kératolytique.

Mais les composés de l'art antérieur présentent également des effets secondaires, qui consistent en des picotements, des tiraillements, des échauffements et des rougeurs désagréables pour l'utilisateur.

On constate donc que subsiste le besoin d'agents antivieillissement ayant une action au moins aussi efficace que celle des composés de l'art antérieur, mais ne présentant pas leurs inconvénients.

L'invention a pour but de pallier les inconvénients des solutions de l'art antérieur et de proposer une composition favorisant la desquamation de la peau et/ou stimulant le renouvellement épidermique, dont l'utilisation n'entraînerait pas de picotements, de tiraillements, d'échauffements ou de rougeurs désagréables pour l'utilisateur.

La demanderesse a trouvé de manière inattendue que l'application d'une quantité efficace d'acide cinnamique ou d'au moins l'un de ses dérivés, permet de favoriser la desquamation de la peau et/ou stimuler le renouvellement épidermique donc de lutter contre le vieillissement.

L'invention a donc pour premier objet l'utilisation en tant que composé actif dans une composition, d'une quantité efficace d'acide cinnamique ou d'au moins l'un de ses dérivés, l'acide cinnamique ou la composition étant destinés à favoriser la desquamation de la peau et/ou stimuler le renouvellement épidermique.

Un autre objet de l'invention est un procédé de traitement non thérapeutique de la peau destiné à favoriser la desquamation de la peau et/ou stimuler le renouvellement épidermique.

L'acide cinnamique est présent sous forme trans dans les huiles essentielles de basilic ou de cannelle, dans la balsamine du Pérou ou encore dans les feuilles de cacao. La forme cis est présente dans l'huile *d'Alpinia malacensis.*

Dans l'art antérieur, l'acide cinnamique ou ses dérivés sont connus pour être utilisés dans des compositions pour la prévention des escarres (JP 07 242 558), comme actif anti-ultraviolets (US 5 093 109), dans des compositions pour permanente (DE 3 301 515, DE 2 912 477, EP 22 996), dans des lotions capillaires (JP 7 053 401, JP 3 041 413), dans des compositions dépigmentantes (JP 5 221 845, JP 1 186 811), comme antioxydant (EP 664 290).

A la connaissance de la demanderesse il n'a jamais été décrit dans l'état de la technique l'utilisation de l'acide cinnamique ou de ses dérivés pour favoriser la desquamation de la peau et/ou stimuler le renouvellement épidermique.

L'invention a donc pour objet l'utilisation en tant que composé actif dans une composition d'une quantité efficace d'acide cinnamique ou d'au moins l'un de ses dérivés, l'acide cinnamique ou la composition étant destinés à favoriser la desquamation de la peau et/ou stimuler le renouvellement épidermique.

L'acide cinnamique ou ses dérivés peuvent être d'origine naturelle ou synthétique. Par origine naturelle, on entend de l'acide cinnamique, ou ses dérivés, préparés à partir de matériel végétal dans lequel ils se trouvent présents à l'état naturel. Par origine synthétique on entend de l'acide cinnamique, ou ses dérivés, préparés par synthèse chimique ou par biotechnologie.
Ainsi, par la suite dans le texte le terme acide cinnamique s'entend comme désignant de l'acide cinnamique, ou ses dérivés, d'origine naturelle ou synthétique, purifiés ou toute préparation les contenant.

Parmi les dérivés d'acide cinnamique utilisables selon l'invention on peut citer à titre d'exemple les esters, les aldéhydes, les alcools, les acides mono- et polyhydroxycinnamiques et dérivés.

Préférentiellement, selon l'invention on utilise l'acide cinnamique.

Bien entendu, il est possible d'utiliser selon l'invention l'acide cinnamique ou ses dérivés seuls ou en mélange.

La quantité d'acide cinnamique, ou de ses dérivés, utilisable selon l'invention, dépend bien évidemment de l'effet recherché et doit être en une quantité efficace pour favoriser la desquamation de la peau et/ou stimuler le renouvellement épidermique et/ou lutter contre le vieillissement cutané intrinsèque et/ou extrinsèque.

A titre d'exemple la quantité d'acide cinnamique ou de l'un de ces dérivés, utilisable selon l'invention peut aller par exemple de 10⁻⁶% à 10% et de préférence de 10⁻³% à 5% du poids total de la composition.

Selon encore un autre aspect, l'invention a pour objet un procédé de traitement non thérapeutique pour favoriser la desquamation de la peau et/ou stimuler le renouvellement épidermique caractérisé en ce qu'on applique sur la peau une composition cosmétique comprenant de l'acide cinnamique ou l'un de ses dérivés.

Ladite composition peut se présenter sous toutes les formes galéniques connues comme par exemple sous la forme d'une émulsion, notamment huile-dans-eau ou eau-dans-huile, voire sous la forme d'une émulsion multiple.

Elle peut également se présenter sous forme d'une solution aqueuse, éventuellement gélifiée, ou sous forme d'une lotion, par exemple biphasée, d'une crème, d'un lait, voire d'une mousse.

La composition utilisée selon l'invention peut comprendre une phase huileuse à base d'huile animale, végétale, minérale, siliconée, fluorée et/ou synthétique.
La phase huileuse peut également comprendre des alcools gras ou des acides gras, ainsi que des tensioactifs.
On peut notamment citer les huiles hydrocarbonées telles que l'huile de paraffine ou de vaseline; le perhydrosqualène; l'huile d'arara, l'huile d'amande douce, de calophyllum, de palme, de ricin, d'avocat, de jojoba, d'olive ou de germes de céréales; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones.
La phase huileuse peut également comprendre une huile démaquillante telle qu'un ester d'acides gras, en particulier les esters obtenus à partir d'un alcool à chaîne droite ou ramifiée ayant de 1 à 17 atomes de carbone et d'un acide gras à chaîne droite ou ramifiée ayant de 3 à 18 atomes de carbone.
Un tel ester peut en particulier être choisi dans le groupe constitué par l'adipate de dioctyle, le palmitate d'éthyl-2 hexyle, l'adipate de diisopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle.
La phase huileuse peut être présente à raison de 5-95% en poids dans le cas d'une émulsion.

La composition utilisée selon l'invention peut comprendre, en outre,
. un agent permettant la mise en suspension de la phase grasse, par exemple un copolymère d'acrylates d'alkyle en C₁₀-C₃₀ et d'acide acrylique ou méthacrylique ou de leur ester (Pemulen TR1, Pemulen TR2, Carbopol 1342 de GOODRICH); ou un copolymère acrylamide/acide méthylpropane sulfonique (Sepigel de SEPPIC), et/ou
. un agent de mise en dispersion de la phase grasse, tel qu'un système émulsionnant ou vésiculaire à base de vésicules, éventuellement de taille nanométrique, constituées de lipides ioniques (liposomes) ou non ioniques, et en particulier les systèmes émulsionnants bien connus de l'homme du métier constitués de stéarate de glycéryl /PEG 100 stéarate (CTFA), d'alcool cétylique et d'alcool stéarylique.

La composition utilisée selon l'invention peut comprendre, en outre, un agent pour modifier sa viscosité, et obtenir des textures plus ou moins gélifiées, tel que :
. les dérivés cellulosiques (carboxyméthylcellulose, hydroxyéthylcellulose, hydroxypropylméthylcellulose),
. les gommes naturelles telles que les gommes de xanthane, de guar, de caroube, les scléroglucanes, les dérivés de chitine ou de chitosane, les carraghénanes,
. les dérivés polycarboxyvinyliques du type Carbomer (vendues par GOODRICH sous les dénominations Carbopol, 940, 951, 980, ou par 3V-SIGMA sous la dénomination Synthalen K ou Synthalen L).

La composition utilisée selon l'invention peut également comprendre, de façon connue, des adjuvants couramment utilisés dans le domaine considéré, tels que les conservateurs, les antioxydants, les parfums, les charges telles que le kaolin ou l'amidon, voire les microsphères creuses, les pigments, les filtres UV, les séquestrants, les huiles essentielles, les matières colorantes, les actifs hydrophiles ou lipophiles tels que les hydratants, notamment la glycérine, le butylène glycol, les anti-inflammatoires comme l'allantoïne, le bisabolol, les anti-radicaux libres comme la vitamine E ou ses dérivés, les apaisants tels que l'eau de bleuet, l'extrait d'iris, les dépigmentants, les actifs biologiques tels que l'urée, les acides aminés, les vitamines et leurs dérivés, les protéines, l'acide salicylique et ses dérivés, les α-hydroxy-acides, l'acide pyrrolidone carboxylique et ses sels, les céramides.
Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition utilisée présente de préférence un pH qui respecte la peau, généralement compris entre 5 et 8, de préférence un pH de 5,5 à 7,5.

Les compositions utilisées selon l'invention peuvent bien entendu être des compositions destinées à un usage cosmétique ou pharmaceutique, particulièrement dermatologique.
De préférence les compositions utilisées selon l'invention sont des compositions à usage topique.

L'invention est illustrée plus en détail dans les exemples suivants.

Exemple 1 : On étudie, dans cet exemple, la capacité de l'acide cinnamique à favoriser la desquamation.
Ce test de screening *in vitro* d'un agent actif sur la desquamation est réalisé sur kératinocytes humains différenciés. Le principe du test repose sur le fait que la desquamation induit la libération de coméocytes. Le pouvoir de desquamation du produit testé va être d'autant plus grand que le nombre de coméocytes libéré sera important.

Le protocole du test a été le suivant : à partir de biopsies de peau humaine, les kératinocytes obtenus par séparation de l'épiderme sont dissociés par action enzymatique à la trypsine et mis en culture à la concentration de 2.10⁵ cellules/ml. La croissance et la différenciation des kératinocytes a été obtenue par culture durant 10 à 20 jours en milieu spécifique. Puis, après élimination du milieu de culture, l'activité du produit à tester est évalué. Pour ce faire, deux prélèvements à T0 et T60 ont été réalisés, c'est-à-dire juste avant l'ajout du produit et 60 minutes après cet ajout. Les prélèvements ainsi effectués ont été analysés au cytomètre de flux pour dénombrer la population de coméocytes. Le cytomètre de flux permet de distinguer les populations de coméocytes et de kératinocytes par traitement à l'acridine orange spécifique de l'ADN des cellules. Cette coloration est spécifique des kératinocytes puisque les coméocytes normaux ne possèdent pas de noyaux donc par d'ADN.

L'indice de détachement cellulaire est déterminé par la différence entre T60 et T0. La même mesure a été réalisée pour un témoin ne contenant pas de produit à tester car les conditions expérimentales induisent inévitablement la libération de coméocytes, même en absence d'actifs.

Le test a été effectué avec de l'acide cinnamique à la concentration de 5.10⁻⁵M.

Les résultats de ces études sont résumés dans le tableau suivant :

| Composés à 5.10⁻⁵ M | % | p** |
|---|---|---|
| Référence* | 96,6 | <0,05 |
| Acide cinnamique | 59,2 | <0,05 |
| Témoin | 0 | - |

| | | |
|---|---|---|
| * Référence : Acide 2-hydroxy-5-octanoylbenzoïque connu comme favorisant la desquamation (Brevet FR-85-06953 de la demanderesse) | | |
| **: p : intervalle de confiance calculé selon la méthode de Dunett. | | |

Les résultats sont donnés en % d'activité par rapport au témoin constitué d'une culture identique en l'absence de composé.
L'activité de l'acide cinnamique sur le détachement cellulaire est donc importante.

Exemple 2 : Exemples de formulations utilisées selon l'invention. Ces compositions ont été obtenues par simple mélange des différents composants.

### Composition 1 : Lait pour le visage

- Huile de vaseline 7,0 g
- Acide cinnamique 2,0 g
- Monostéarate de glycéryle, stéarate de polyéthylène glycol (100 OE) 3,0 g
- Polymère carboxyvinylique 0,4 g
- Alcool stéarylique 0,7 g
- Protéines de soja 3,0 g
- NaOH 0,4 g
- Conservateur qs
- Eau qsp 100 g

Cette composition se présente sous forme d'un lait pour le visage, ayant de bonnes propriétés cosmétiques, et étant doux et confortable à utiliser.
Le pH de la composition est d'environ 5,5.

### Composition 2 : Lotion

- Acide cinnamique 0,5 g
- Palmitate d'éthyl-2 hexyle 10,0 g
- Cyclopentadiméthylsiloxane 20,0 g
- Butylène glycol 5,0 g
- Conservateur qs
- Eau qsp 100g

Cette lotion, qui ne contient pas de tensioactif, favorise la desquamation de la peau.

### Composition 3 : Lait

- Palmitate d'octyle 35,0 g
- Glycérine 2,0 g
- Acide cinnamique 2,0g
- Polymère réticulé acrylates C10-C30/alkylacrylates 0,1 g
- Triéthanolamine 0,1 g
- Acides aminés de blé 1,0 g
- Conservateur qs
- Eau qsp 100 g

Le lait obtenu, qui ne contient pas de tensioactif, possède de bonnes propriétés cosmétiques.

### Composition 4 : Gel pour le visage

- Glycérine 10,0 g
- Acide cinnamique 1,0 g
- Cocoamphodiacétate de disodium 1,0 g
- Conservateur qs
- Eau qsp 100 g

Le gel obtenu possède de bonnes propriétés cosmétiques.

### Composition 5 : Gel nettoyant à l'eau

- Butylène glycol 7,0 g
- Sarcosinate de lauroyl sodium 4,0 g
- Acide cinnamique 0,5 g
- Triéthanolamine 0,8 g
- Carbomer 0,5 g
- Conservateur qs
- Eau qsp 100 g

Le gel obtenu possède de bonnes propriétés cosmétiques.

## Revendications

1. Utilisation en tant que composé actif dans une composition, d'une quantité efficace d'acide cinnamique ou de l'un de ses dérivés, l'acide cinnamique ou la composition étant destinés à favoriser la desquamation de la peau et/ou stimuler le renouvellement épidermique.

2. Utilisation selon la revendication précédente, **caractérisée par le fait que** le dérivé d'acide cinnamique est choisi parmi les esters, les aldéhydes, les alcools, les acides mono et polyhydroxycinnamiques et dérivés.

3. Utilisation selon l'une quelconque des revendications précédentes, **caractérisée par le fait que** l'acide cinnamique ou l'un de ses dérivés, est utilisé en une quantité allant de 10⁻⁶% à 10% du poids total de la composition.

4. Utilisation selon l'une quelconque des revendications précédentes, **caractérisée par le fait que** l'acide cinnamique ou l'un de ses dérivés, est utilisé en une quantité allant de 10⁻³% à 5% du poids total de la composition. du poids total de la composition

5. Utilisation selon l'une quelconque des revendications précédentes, dans une composition se présentant sous la forme d'une émulsion, d'une solution aqueuse, éventuellement gélifiée, d'une lotion notamment biphasée, d'une crème, d'un lait, d'une mousse.

6. Procédé de traitement non thérapeutique pour favoriser la desquamation de la peau et/ou stimuler le renouvellement épidermique **caractérisé en ce qu'**on applique sur la peau une composition cosmétique comprenant comme agent actif une quantité efficace d'acide cinnamique ou de l'un de ses dérivés.

## Patentansprüche

1. Verwendung einer wirksamen Menge von Zimtsäure oder mindestens einem ihrer Derivate als Wirkstoff in einer Zusammensetzung, wobei die Zimtsäure oder die Zusammensetzung zur Förderung der Abschuppung der Haut und/oder zur Stimulierung der Epidermiserneuerung vorgesehen sind.

2. Verwendung nach dem vorhergehenden Anspruch, **dadurch gekennzeichnet, dass** das Zimtsäurederivat unter den Estern, Aldehyden, Alkoholen, Mono- und Polyhydroxyzimtsäuren und deren Derivaten ausgewählt ist.

3. Verwendung nach einem der vorhergehenden Ansprüche, **dadurch gekennzeichnet, dass** die Zimtsäure oder eines ihrer Derivate in einer Menge von 10⁻⁶ bis 10 % des Gesamtgewichts der Zusammensetzung verwendet wird.

4. Verwendung nach einem der vorhergehenden Ansprüche, **dadurch gekennzeichnet, dass** die Zimtsäure oder eines ihrer Derivate in einer Menge von 10⁻³ bis 5 % des Gesamtgewichts der Zusammensetzung verwendet wird.

5. Verwendung nach einem der vorhergehenden Ansprüche in einer Zusammensetzung, die in Form einer Emulsion, einer wässrigen Lösung, die gegebenenfalls in ein Gel übergeführt ist, einer Lotion, insbesondere zweiphasigen Lotion, einer Creme, einer Milch oder eines Schaums vorliegt.

6. Nicht therapeutisches Verfahren zur Förderung der Abschuppung der Haut und/oder Stimulierung der Epidermiserneuerung, **dadurch gekennzeichnet, dass** auf die Haut eine kosmetische Zusammensetzung aufgetragen wird, die als Wirkstoff Zimtsäure oder eines ihrer Derivate in einer wirksamen Menge enthält.

## Claims

1. Use, as an active compound in a composition, of an effective amount of cinnamic acid or of one of its derivatives, the cinnamic acid or the composition being intended to promote desquamation of the skin and/or to stimulate epidermal renewal.

2. Use according to the preceding claim, **characterized in that** the cinnamic acid derivative is chosen from mono- and polyhydroxycinnamic acids, alcohols, aldehydes, esters and derivatives.

3. Use according to either of the preceding claims, **characterized in that** the cinnamic acid, or one of its derivatives, is used in an amount ranging from 10⁻⁶% to 10% of the total weight of the composition.

4. Use according to any one of the preceding claims, **characterized in that** the cinnamic acid, or one of its derivatives, is used in an amount ranging from 10⁻³% to 5% of the total weight of the composition.

5. Use according to any one of the preceding claims, in a composition in the form of an emulsion, an aqueous solution, which may be gelled, a lotion, in particular a two-phase lotion, a cream, a milk or a mousse.

6. Non-therapeutic treatment process for promoting desquamation of the skin and/or stimulating epidermal renewal, **characterized in that** a cosmetic composition comprising, as active agent, an effective amount of cinnamic acid or one of its derivatives, is applied to the skin.
